(19) Europäisches Patentamt
European Patent Office
Office européen des brevets

(11) Publication number: 0 270 692 A1

# (12) EUROPEAN PATENT APPLICATION

published in accordance with Art. 158(3) EPC

(21) Application number: 87904125.9

(22) Date of filing: 26.06.87

Data of the international application taken as a basis:

(86) International application number: PCT/JP 87/00432

(87) International publication number: WO 88/00049 (14.01.88 88/2)

(51) Int. Cl.4: A 61 K 31/435, C 07 D 491/044

(30) Priority: 26.06.86 JP 150041/86

(43) Date of publication of application: 15.06.88 Bulletin 88/24

(84) Designated Contracting States: AT BE CH DE FR GB IT LI LU NL SE

(71) Applicant: KYOWA HAKKO KOGYO CO., LTD., Ohtemachi Bldg., 6-1 Ohtemachi I-chome, Chiyoda-ku Tokyo 100 (JP)

(72) Inventor: KUMAZAWA, Toshiaki, 1194-83, Shimotogari, Nagaizumi-cho, Sunto-gun Shizuoka 411 (JP)
Inventor: OBASE, Hiroyuki, 1188, Shimotogari, Nagaizumi-cho, Sunto-gun Shizuoka 411 (JP)
Inventor: NITO, Masaaki, 190-3, Nagaoka Izunagaoka-cho, Tagata-gun, Shizuoka 410-22 (JP)
Inventor: SANO, Tomoyuki, 1188, Shimotogari, Nagaizumi-cho, Sunto-gun Shizuoka 411 (JP)
Inventor: KUBO, Kazuhiro, 625-11, Kashiwakubo Shuzenji-cho, Tagata-gun Shizuoka 410-24 (JP)

(74) Representative: Vossius & Partner, Siebertstrasse 4 P.O. Box 86 07 67, D-8000 München 86 (DE)

## (54) ANTIARRHYTHMIC AGENT.

(57) An antiarrhythmic agent which contains a [1]benzoxepino[3,4-b]pyridine derivative or pharmacologically acceptable acid addition salt thereof as effective ingredient and a method for treatment or prophylaxis of arrhythmia.

EP 0 270 692 A1

EP 87 90 4125.9

00270692

SPECIFICATION

# ANTIARRHYTHMIC AGENT

## Technical Field

The present invention relates to an antiarrhythmic agent containing a [1]benzoxepino[3,4-b]pyridine derivative or pharmacologically acceptable acid addition salt thereof as effective ingredient.

## Background Art

Heretofore, various compounds have been known as the [1]benzoxepinopyridine derivative and [1]benzthiepinopyridine derivative. For example, as the compound having antiinflammatory and analgesic activities, a compound represented by the formula:

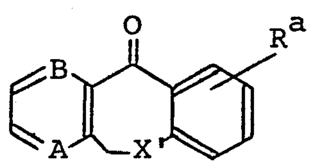

wherein A and B are different from each other and represent -CH- or N, X' means oxygen or sulfur and $R^a$ represents hydrogen or lower alkyl is disclosed in JP-A-117496/1974 and Chem. Pharm. Bull. Vol.29; page 3515 (1981).

A compound represented by the formula

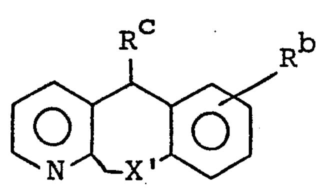

wherein $R^b$ represents hydrogen, alkyl, halogenated alkyl, alkoxy or halogen, X' represents oxygen or sulfur, $R^c$ represents $Z'-N(-)-(CH_2)_{n'}-Y'$ or 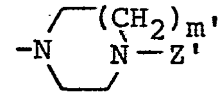 wherein Y' represents amino, substituted amino, heterocyclic group or substituted heterocyclic group, Z' represents hydrogen, alkyl or acyl, n' represents an integer of 1 to 3 and m' is 0, 1 or 2 is described in JP-A-6690/1985, JP-A-89419/1985, USP 4547496 and EP 129879A2.

A compound represented by the formula:

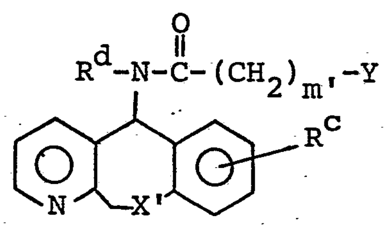

wherein $R^c$, X', Y' and m' have the same meanings as previously defined and $R^d$ represents hydrogen or alkyl is described in JP-A-78985/1985 as the compound having untiulcer activity.

However, there have been no reports about [1]-benzoxepinopyridine derivative or [1]benzthiepinopyridine derivative having antiarrhythmic activity. The known compounds having antiarrhythmic activity are not structurally analogous to the derivatives described above.

Pirmenol represented by the following formula:

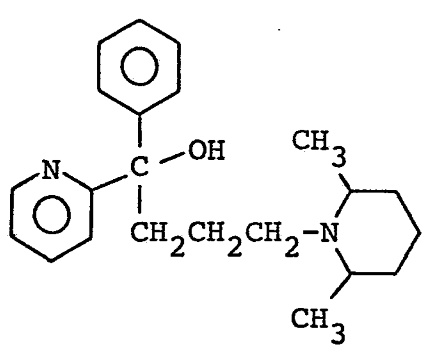

is known as the compounds having a pyridine skeleton and antiarrhythmic activity (USP 4,112,103).

## Disclosure of the Invention

The pharmaceutical composition having an excellent antiarrhythmic activity of the present invention contains, as effective ingredient, [1]benzoxepino[3,4-b]pyridine derivative represented by formula (I) or a pharmacologically acceptable acid addition salt thereof.

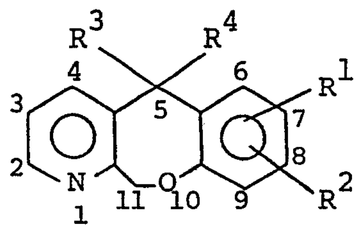

In the formula,

a) $R^1$ represents lower alkoxy, halogenated lower alkyl, halogenated lower alkoxy, hydroxy, lower alkylamino, di-lower alkylamino, cycloalkylamino, lower alkanoylamino, carboxyamino or lower alkoxycarbonylamino,

$R^2$ represents hydrogen,

$R^3$ represents $Z-N-(CH_2)_n-Y$ or 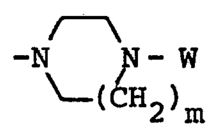

wherein Y represents amino, lower alkylamino, di-lower alkylamino, cycloalkylamino, heterocyclic group containing nitrogen or lower alkyl-substituted heterocyclic group containing nitrogen,

Z represents hydrogen, lower alkyl or lower alkanoyl

n represents 1, 2 or 3,

W represents hydrogen or lower alkyl, and

m represents 0, 1 or 2, and

$R^4$ represents hydrogen or

b) $R^1$ represents lower alkoxy or halogenated lower alkyl,

00270692

$R^2$ represents hydrogen,
$R^3$ represents $-NHCOCH_2Y$ wherein Y has the same meaning as previously defined, and
$R^4$ represents hydrogen, or

c) $R^1$ and $R^2$ simultaneously represent lower alkoxy,
$R^3$ represents $Z-\underset{|}{N}-(CH_2)_n-Y$, 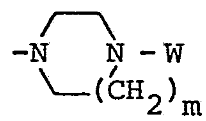 or $-X-(CH_2)_n-Y$
wherein n, Y, Z, m and W have the same meanings as previously defined, and X represents oxygen or sulfur, and
$R^4$ represents hydrogen or

d) $R^1$ represents hydrogen, lower alkyl halogen, halogenated lower alkyl, lower alkoxy, halogenated lower alkoxy, hydroxy, lower alkylamino, di-lower alkylamino, cycloalkylamino, lower alkanoylamino, carboxyamino or lower alkoxycarbonylamino,
$R^2$ represents hydrogen,
with respect of $R^3$ and $R^4$,

(1) $R^3$ represents $-X-(CH_2)_n-Y$ wherein n, X and Y have the same meanings as previously defined, and $R^4$ represents hydrogen or
(2) $R^3$ and $R^4$, taken together, are $=CH(CH_2)_nY$ wherein n and Y have the same meanings as previously defined, or
(3) $R^3$ represents $-(CH_2)_{n+1}-Y$ wherein n and Y have the same meanings as previously defined and $R^4$ represents cyano or carbamoyl.

[hereinafter referred to as Compound (I), and the compounds with the other formula numbers are likewise referred to]

In the definitions of groups in formula (I), the lower alkyl includes straight or branched chain alkyl having 1 - 6 carbon atoms such as methyl, ethyl, propyl, isopropyl, etc., the lower alkoxy includes straight or branched chain alkoxy having 1 to 6 carbon atoms such as methoxy, ethoxy, propoxy, isopropoxy, etc., and the halogenated lower alkyl includes straight or branched chain

alkyl having 1 - 3 carbon atoms substituted by 1 to 3 and the same or different halogen atoms such as fluorine chlorine and bromine, including for example, trifluoromethyl, etc. The lower alkylamino includes alkylamino having straight or branched chain alkyl moiety having 1 - 6 carbon atoms, such as methylamino, ethylamino, isopropylamino, etc. and the di-lower alkylamino includes amino substituted by the same or different and straight or branched chain alkyl moiety having 1 - 6 carbon atoms, such as dimethylamino, diethylamino, diisopropylamino, etc. The cycloalkylamino includes cycloalkylamino having 5 - 7 carbon atoms such as cyclopenthylamino, cyclohexylamino, etc. and the halogenated lower alkoxy includes alkoxy having 1 - 6 carbon atom(s) substituted by 1 to 3 and the same or different halogen atom such as fluorine, chlorine and bromine, including, for example, trifluoromethoxy. 2,2,2-trifluoroethoxy, etc. The lower alkoxycarbonylamino includes alkoxycarbonylamino having 2 - 7 carbon atoms and straight or branched chain alkyl moiety including, for example, methoxycarbonylamino, ethoxycarbonylamino, etc.

The heterocyclic group containing nitrogen includes single or fused double membered heterocyclic group having 5 - 8-membered rings and 1 - 3 nitrogen atoms as the hetero atom; and single or fused double membered heterocyclic group having 5 - 8-membered rings and 1 - 3 nitrogen atoms and one oxygen or sulfur as the hetero atom; such as pyrrolidino, pyrrolidinyl, piperidino, piperidyl, piperazino, piperazinyl, homopiperazino, homopiperazinyl, pyridyl, pyrrolyl, quinuclidinyl, imidazolyl, morpholino, etc.

The lower alkyl substituted-heterocyclic group containing nitrogen includes the same heterocyclic group containing nitrogen atom as described above except that it has alkyl substituents, such as 1-ethyl-2-pyrrolidinyl, 4-methyl-1-piperazinyl, 2,6-dimethylpiperidino, etc., the substituents being 1 - 3, the same or different and straight or branched chain alkyls having 1 - 6 carbon atoms such as

methyl, ethyl, isopropyl, etc.

The lower alkanoyl includes straight or branched chain alkanoyl having 1 - 6 carbon atoms including, for example, formyl, acetyl and propionyl. The halogen includes fluorine, chlorine, bromine and iodine.

The lower alkanoylamino includes straight or branched chain alkanoylamino having 1 - 6 carbon atoms including, for example, formylamino, acetylamino, etc.

The pharmacologically acceptable acid addition salt of Compound I include acid addition salt with pharmacologically acceptable inorganic acid such as hydrochloric acid, hydrobromic acid, sulfuric acid, phosphoric acid and nitric acid and with pharmacologically acceptable organic acid such as acetic acid, benzoic acid, maleic acid, fumaric acid, succinic acid, tartaric acid, citric acid, oxalic acid, methanesulfonic acid, toluenesulfonic acid, aspartic acid and glutamic acid.

The test results on acute toxicity and antiarrhythmic activity of representative compounds of Compound I are shown below.

Test for Acute Toxicity

Groups of 5 male dd-strain mice weighing 20 ±1 g were used. The test compounds were administered either orally (0.3 mg/kg) or intraperitoneally (0.1 mg/kg).

The MLD (minimum lethal dose) was determined by observing the mortality for 7 days after the administration. Results are given in Table 1.

Test for Antiarrhythmic Activity

Antiarrhythmic activity was examined according to mouse chloroform-induced ventricular arrhythmias test.

DD-strain mice weighing 18 - 22 g were abstinent from food and water for 4 - 5 hours and antiarrhythmic activity was examined according to the Block's method [Life Science 28, 2623 (1981)]. Drugs tested were orally

administered (100 mg/kg) and the mice were exposed to chloroform. After stop of respiration, electrocardiogram was measured according to standard limb leading method. The case where 720 or less heart beat/min was maintained for 30 seconds was regarded as effective.

The test compound was judged as being effective when two or more examples were effective among three examples.

The results are shown in Table 1.

Table 1

| Compound *1 | Acute Toxicity MLD | | Anti-arrhythmic Activity |
|---|---|---|---|
| | p.o. | i.p. | |
| 1.5 Fumarate of Compound 1 | more than 300 mg/kg | more than 100 mg/kg | Effective |
| Trihydrochloride monohydrate of Compound 4 | " | " | " |
| Trihydrochloride monohydrate of Compound 8 | " | " | " |
| Trihydrochloride monohydrate of Compound 13 | " | " | " |
| Trihydrochloride monohydrate of Compound 14 | " | " | " |
| Compound 16 | " | " | " |
| Dihydrochloride of Compound 23 | " | " | " |
| 5-[2-diethylamino)-ethyl]amino-7-methyl-5,11-dihydro[1]benzoxepino[3,4-b]pyridine *2 | - | - | *3 Ineffective |

*1 The Compound corresponding to the compound number is designated in Table 3

*2 Compound obtained in Example 2 of JP-A-6690/85

*3 All of the 3 examples are ineffective.

A compound represented by formula (I-1) among Compound I

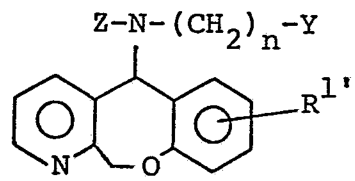

(I-1)

wherein Z, Y and n have the same meanings as defined in formula (1) and $R^{1'}$ represents lower alkoxy is good as the antiarrhythmic agent and above all, a compound wherein $R^{1'}$ is bound to 7-position is excellent, because Compound (I-1) has a good antiarrhythmic activity and a less toxicity, and the antiarrhythmic doses of Compound (I-1) used in a variety of arrhythmia models are very apart of those of adverse effects.

The antiarrhythmic activity, the acute toxicity and the results of the representative adverse effect tests using 1.5 fumarate of Compound 1 are shown in Table 2. When 500 mg/kg of the compound was orally administered to a male dd-strain mouse, no effect on central nerve such as central excitatory state, central inhibitory state or muscle reluxing state was observed.

In Table 2, Test Nos. 1 and 4 are tests on antiarrhythmic activity and Test No. 3, on anticholine activity and Test No. 5, on side effect. What is meant by the small ratio of Test No. 1 to that of Test No. 3 and the small ratio of Test No. 4 to that of Test No. 5 is that the compound has high therapeutic ratio (wide safety margin).

Table 2

| | | |
|---|---|---|
| 1 | Chloroform-induced ventricular antiarrhythmic activity test *1 $ED_{50}$ | 80 mg/kg |
| 2 | Acute toxicity test *2 $LD_{50}$ iv<br>po | 93 mg/kg<br>>1000 mg/kg |
| 3 | Mydriasis activity test *3 $ED_{100}$ | 235 mg/kg |
| 4 | The concentration required to prolong effective refractory period by 30% *4 | 4.9 μg/ml |
| 5 | The concentration required to reduce the amplitude of myocardial contractile force by 30% *4 | 110 μg/ml |
| 6 | 5/4 (ratio) | 22.4 |

*1 The test was conducted according to the foregoing mouse chloroform-induced venticular antiarrhythmic activity test to determine $ED_{50}$.

*2 The test was conducted according to the foregoing acute toxicity test. The test compound was orally (po) or intravenously (iv) administered and $LD_{50}$ was calculated from the number of death in 72 hours after administration.

*3 Male dd-strain mice weighing 18 - 22 g were used. Under the illumination of 1000 luxes, pupil size was measured with stereoscopic microscope and the mice, the pupil size of which is 0.2 ± 0.1 mm, were used. The pupil size was measured before and 30 minutes after the oral administration of test compound and $ED_{100}$ was defined as a dose required to double the size.

*4 Male Hartley-strain guinea pigs weighing 300 - 500 g were used. Immediately after beating to death, the breast was cut open, and the left atrium excised was hung up in 30 ml of Krebs-Henseleit solution (31 ± 1°C, under

95% $O_2$ + 5% $CO_2$ gas) under load of 0.5 g. The contractile force formed by electric stimulus of square wave pulses (2Hz, 1 msec, 1.5 times of threshold voltage) was recorded in polygraph through tension transducer. Effective refractory period was obtained by the Vaugham-Williams and Szekers method [Br. J. Pharmacol. 17, 424 (1961)]. Stimulus frequency was continuously increased from 2Hz. The maximal driven frequency was determined by progressive elevation of stimulation frequency until the tissue failed to follow each stimulus, thus effective refractory period being calculated as the reciprocal of the maximal driven frequency. That is, 3Hz, 4Hz and 5Hz are defined as 333 msec, 250 msec and 200 msec, respectively. After twice control measurements were carried out, the test compound was added to a nutrient medium and ten minutes later, the value was measured and compared to the control. Concurrently, the effect against myocardial contractile force was examined. The concentration required to prolong effective refractory period by 30% and the concentration required to reduce the amplitude of myocardial contractile force by 30% were determined.

Compound (I) and the pharmacologically acceptable acid addition salt thereof can be used in various medicament forms for the administration purposes in view of the pharmacological activity thereof.

The present medicament composition can be prepared by uniformly mixing an effective amount of Compound (I) or an acid addition salt thereof as an active component with a pharmaceutically acceptable carrier or excipient. The carrier can take a wide range of forms in accordance with a desirable medicament form for the administration. These medicament compositions are desirably in a unit dosage form suitable for the oral administration or injection administration. In the preparation of a composition in the oral dosage form, any useful, pharmaceutically acceptable carrier can be used. For example, an oral liquid preparation such as a suspended medicament or syrup

medicament can be prepared using water; sugars such as sucrose, sorbitol, fructose, etc.; glycols such as polyethylene glycol, propylene glycol, etc.; oils such as sesame oil, olive oil, soybean oil, etc.; antiseptics such as alkyl parahydroxybenzoate, etc.; and flavors such as strawberry flavor, peppermint, etc. Powder, pills, capsules and tablets can be prepared using an excipient such as lactose, glucose, sucrose, mannitol, etc.; a disintegrator such as starch, sodium alginate, etc.; a lubricant such as magnesium stearate, talc, etc.; a binder such as polyvinyl alcohol, hydroxypropylcellulose, gelatin, etc.; a surfactant such as fatty acid esters; and a plasticizer such as glycerine, etc. Tablets and capsules are the most useful, oral unit dosage forms because of easy administration. To prepare tablets and capsules, solid carriers for medicament are used. Injection solution can be prepared using a carrier consisting of distilled water, a salt solution, a glucose solution or a mixture of the salt solution and the glucose solution. The effective dosage of Compound I or salt thereof is 150 - 800 mg / adult (60 kg) / day for oral administration and the number of administration is preferrably 3 times/day.

The effective dosage is 150 - 600 mg/adult (about 60 kg) for non oral-administration, at the time when the administration is required.

The treatment, prophylaxis or prevention of arrhythmias such as sinoatrial arrhythmias, sinus tachycardia, supra ventricular arrhythmias and ventricular arrhythmias can be made by administration of Compound (I).

The process for producing Compound I is explained below.

Compound I can be produced according to the method described below using Compound II represented by the formula:

0270692

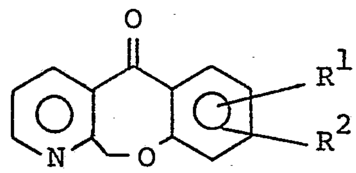

( II )

wherein $R^1$ and $R^2$ have the same meanings as defined in formula (I)

Compound II can be produced according to the methods described in JP-A-117496/74 or Chem. Pharm. Bull. <u>29</u>, 3515 (1981) or according to the methods shown in Compound production examples 33 and 35.

<u>Process A</u>

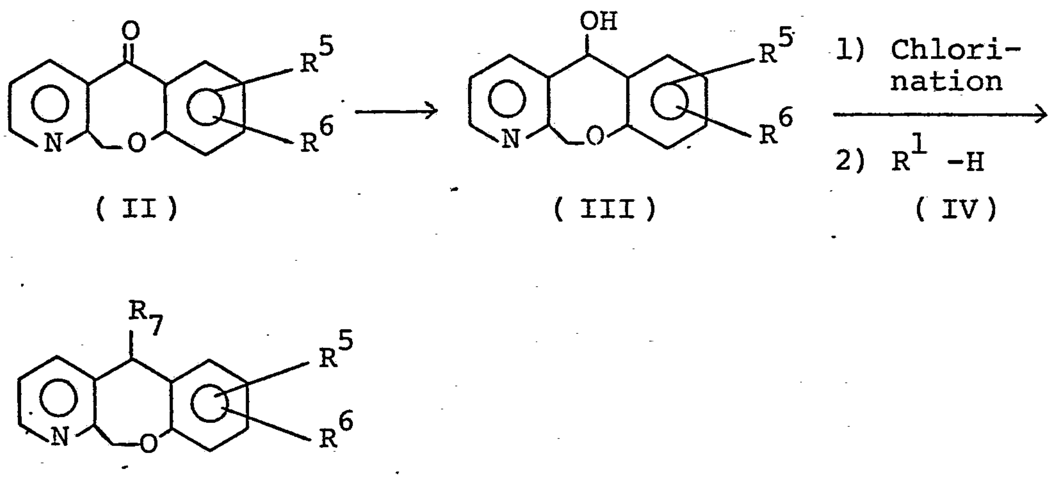


( II ) ( III ) ( IV )

( I-1 )

wherein $R^5$ represents hydrogen, lower alkyl, halogen, halogenated lower alkyl, lower alkoxy, halogenated lower alkoxy, lower alkylamino, di-lower alkylamino, cycloalkylamino, lower alkanoylamino, carboxylamino or lower alkoxycarbonylamino and $R^6$ represents hydrogen or $R^5$ and $R^6$ simultaneously represent lower alkoxy;

$R^7$ represents $Z'-N-(CH_2)_n-Y$, 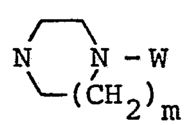 or $-X-(CH_2)_n-Y$

wherein n, Y, W, m and X have the same meanings as defined in formula (I) and Z' is hydrogen or lower alkyl;

Compound II is subjected to reaction in an alcoholic solvent such as methanol and ethanol in addition of tetrahydrofuran in the presence of 0.5 - 1 equivalent of sodium borohydride at from 0°C to room temperature for 1 - 6 hours to obtain Compound III. Compound III is subjected to reaction with 1 - 2 equivalents of halogenating agent such as thionyl chloride in dichloromethane under from ice cooling to room temperature for 1 - 6 hours to obtain chlorinated Compound III. The mixture is subjected to reaction with 1 - 5 equivalents of the compound represented by the formula $R^7$-H (IV) if necessary, in the presence of 1 - 5 equivalents of a base such as pyridine, triethylamine, etc., in dichloromethane under ice cooling to room temperature for 1 - 6 hours to obtain Compound I-1. The production example of Compound III is shown in Compound production example 36.

Process B

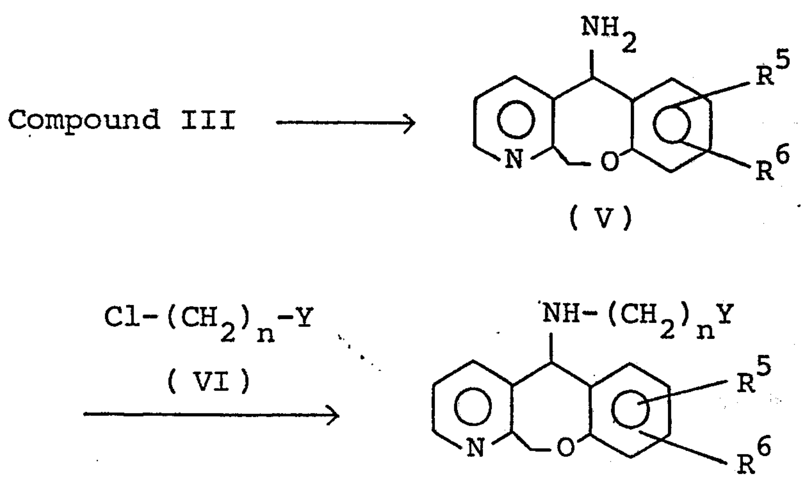


( V )

( I-2 )

In the formulae, $R^5$ and $R^6$ have the same meanings as defined in formula (II), and Y and n have the same meanings as defined in formula (I).

0020692

After chlorination of Compound III with thionyl chloride, in the similar manner to in Process A, the mixture is subjected to reaction with an excess amount of aqueous ammonia solution such as dichloromethane ammonia solution under ice cooling to room temperature for 1 - 6 hours to obtain Compound V. Compound V is subjected to reaction with 1 - 2 equivalents of $Cl-(CH_2)_n-Y$ (Compound VI) or hydrochloride thereof wherein n and Y have the same meanings as defined in formula (1), if necessary, in the presence of a base such as 1 - 5 equivalents of triethylamine and a catalytic amount of potassium iodide or sodium iodide in an inert solvent such as toluene acetonitrile, etc., at a temperature of from room temperature to the boiling point of solvent to be utilized for 4 - 20 hours to obtain Compound I-2.

The production of Compound V is exemplified in Compound production example 24.

<u>Process C</u>

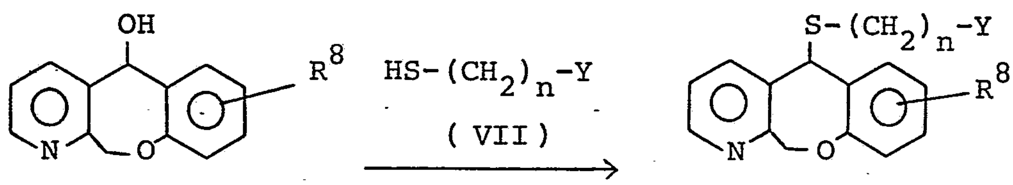


(III')  (I-3)

In the formulae, $R^8$ represents hydrogen, lower alkyl, halogen, halogenated lower alkyl, lower alkoxy, halogenated lower alkoxy, lower alkylamino, di-lower alkylamino, cycloalkylamino, lower alkanoylamino, carboxyamino or lower alkoxycarbonylamino and Y and n have the same meanings as defined in formula (I).

Compound III' is subjected to reaction with 1 - 2 equivalents of Compound (VII) or acid addition salt thereof in the presence of 1 - 2 equivalents of trifluoroacetic anhydride on the basis of Compound III in an inert solvent such as carbon tetrachloride, chloroform, 1,2-dichloroethane,

1,1,2,2-tetrachloroethane, etc., at a temperature of from room temperature to the boiling point of the solvent to be utilized for 4 - 20 hours to obtain Compound I-3.

Process D

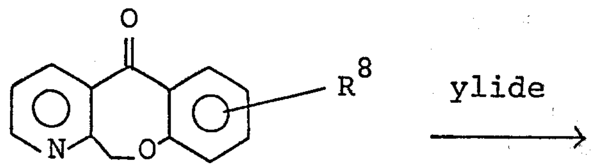


( II' )

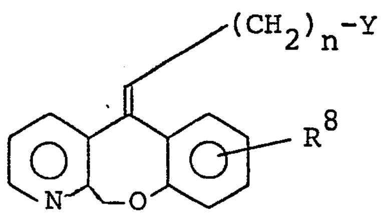

( I-4 )

+

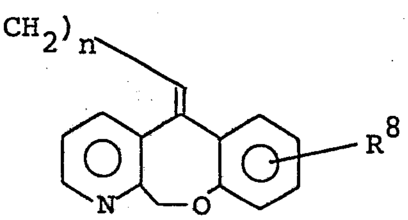

( I-5 )

In the formulae, $R^8$, Y and n have the same meaning as defined in formulae (III') and (I).

Ylide having the formula $Ph_3P = CH(CH_2)_n-Y$ wherein n and Y have the same meanings as previously defined, can be produced by incubating 1 - 5 equivalents of $Ph^+_3P-(CH_2)_{n+1}-Y \cdot Br^-$ (Compound VIII) or the acid addition salt thereof on the basis of Compound II', wherein n and Y have the same meanings as previously defined, in the presence of 2 equivalents of a base on the basis of Compound VIII such as sodium hydride and n-butyllithium in an organic solvent such as ether, tetrahydrofuran, 1,2-dimethoxyethane, dimethyl sulfoxide, dimethylformamide, etc., at a temperature of from 0°C to room temperature for 1 to 5 hours.

To the ylide reaction mixture is added one equivalent of Compound (II') and the mixture is subjected to reaction at a temperature of -78°C to room temperature for 2 - 12 hours to obtain Compounds I-4 and I-5. The reaction mixture is subjected to column chromatography on silica gel

or recrystallization for the isolation of the two compounds from each other.

Process E

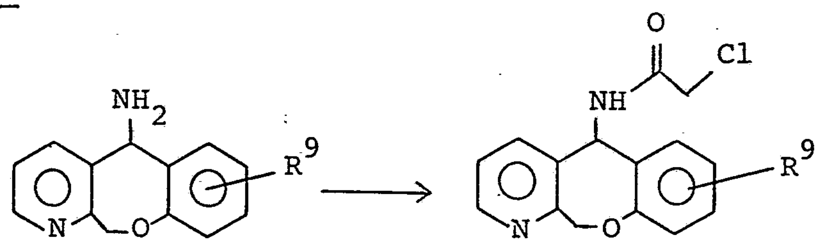

( V' ) ( IX )

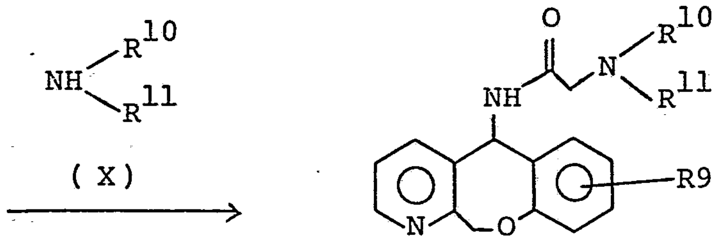

( X )

( I-6 )

In the formulae, $R^9$ represents lower alkoxy, halogenated lower alkoxy or halogenated lower alkyl and $R^{10}$ and $R^{11}$ are the same or different and independently represent hydrogen, lower alkyl or cycloalkyl, or the whole of 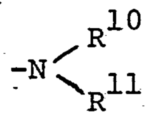 represents the heterocyclic ring or the lower alkyl-substituted heterocyclic ring, contained in the definition of Y.

Compound V' obtained in Process B is subjected to reaction with 1 - 1.5 equivalents of α-chloroacetyl-chloride, if necessary, in the presence of 1 - 2 equivalents of a base such as pyridine and triethylamine in an organic solvent such as dichloromethane, ethylether, etc. under ice cooling or at room temperature for 1 - 3 hours to obtain Compound IX.

Compound IX is subjected to reaction with 1 - 5 equivalents of Compound X, if necessary, in the presence of a catalytic amount of sodium iodide or potassium iodide in an organic solvent, such as toluene, acetonitrile, etc.

at a temperature of from room temperature to the boiling point of the solvent to be utilized for 1 - 6 hours to obtain Compound I-6.

Process F

Compound III'

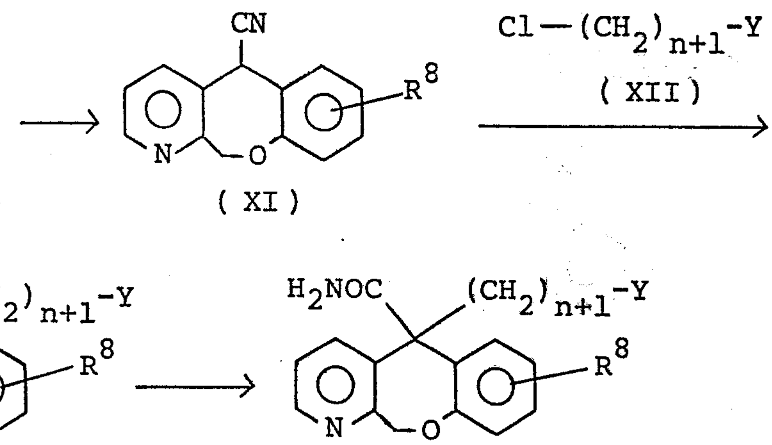


( XI )

( I-7 )

( I-8 )

In the formulae, $R^8$, Y and n have the same meanings as defined in formula (III') and formula (I).

After chlorination of Compound III' with thionyl chloride according to the similar manner to in Process A, the mixture is subjected to reaction with 1 - 3 equivalents of cyanide such as sodium cyanide, potassium cyanide, copper cyanide, silver cyanide, etc. in an organic solvent such as acetonitrile, dimethylformamide, etc. at a temperature of from room temperature to the boiling point of the solvent to be utilized for 1 - 3 hours to obtain Compound XI.

Compound XI is subjected to reaction with 1 - 1.5 equivalents of a base such as sodium hydride and sodium amide in an organic solvent such as toluene, tetrahydrofuran, dimethylformamide, etc. at a temperature of from room temperature to the boiling point of the solvent to be utilized for 1 - 3 hours to form an anion. To the reaction mixture is added 1 - 2 equivalents of Compound XII and the mixture is subjected to reaction at a temperature of from room temperature to the boiling point of the solvent to be utilized for 3 - 10 hours to obtain Compound I-7.

Compound I-7 is subjected to hydrolysis under acidic or basic condition, for example, by heating it in an mineral acid such as concentrated sulfuric acid or concentrated hydrochloric acid to obtain Compound I-8

Process G

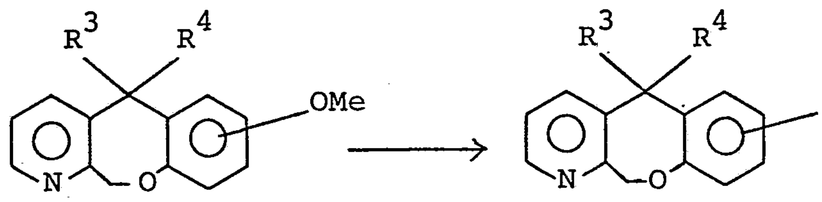

( I-9 ) ( I-10 )

In the formulae, $R^3$ and $R^4$ have the same meanings as defined in formula (I).

Compound I-9 obtained according to Processes A - F is subjected to reaction with 1 - 3 equivalents of boron halogenide such as boron trichloride, boron tribromide, boron triiodide, etc. in an organic solvent such as dichloromethane at a temperature of from -78°C to room temperature for 2 - 12 hours and the mixture is subjected to hydrolysis by adding water to obtain Compound I-10.

Compound I obtained according to Processes A - G is in a form of crystals or oily matter as free base, and can be purified by column chromatography or recrystallization to obtain Compound I in highly purified form.

A pharmacologically acceptable acid addition salt of Compound I can be produced by reacting Compound I with an acid according to the conventional manner.

Among Compound I, the compound represented by formula I-11 and a pharmacologically acceptable acid addition salt thereof are novel compounds.

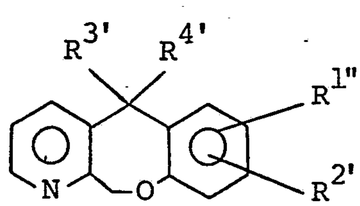

( I-11 )

In the formula:

a) $R^{1''}$ represents halogenated lower alkoxy, hydroxy, lower alkylamino, di-lower alkylamino, cycloalkylamino, lower alkanoylamino, carboxyamino or lower alkoxycarbonylamino;

$R^{2'}$ represents hydrogen;

$R^{3'}$ represents $Z-\underset{|}{N}-(CH_2)_n-Y$ or 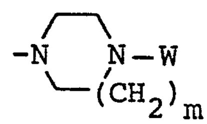

wherein Y represents amino, lower alkylamino, di-lower alkylamino, cycloalkylamino, heterocyclic group containing nitrogen or lower alkyl-substituted heterocyclic group containing nitrogen;

Z represents hydrogen, lower alkyl or lower alkanoyl,

n represents 1, 2 or 3;

W represents hydrogen or lower alkyl;

m represents 0, 1 or 2 and

$R^{4'}$ represents hydrogen;

b) $R^{1''}$ and $R^{2'}$, taken together, are lower alkoxy;

$R^{3'}$ represents $Z-\underset{|}{N}-(CH_2)_n-Y$, 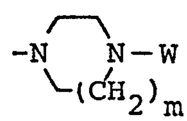 or $-X-(CH_2)_n-Y$

wherein n, Y, Z, m and W have the same meanings as previously defined and X is oxygen or sulfur and

$R^{4'}$ represents hydrogen or,

c) $R^{1''}$ represents hydrogen, lower alkyl, halogen, halogenated lower alkyl, lower alkoxy, halogenated lower alkoxy, hydroxy, lower alkylamino, di-lower alkylamino, cycloalkylamino, lower alkanoylamino, carboxylamino or lower alkoxycarbonylamino;

$R^{2'}$ represents hydrogen;

With respect to $R^{3'}$ and $R^{4'}$

(1) $R^{3'}$ represents $-X-(CH_2)_n-Y$ wherein n, X and Y have the same meanings as previously defined, $R^{4'}$ represents hydrogen;

(2) $R^{3'}$ and $R^{4'}$, taken together, are $=CH_2(CH_2)_nY$ wherein n and Y have the same meanings as previously defined or

(3) $R^{3'}$ represents $-(CH_2)_{n+1}-Y$ wherein n and Y have the same meanings as previously defined and $R^{4'}$ represents cyano or carbamoyl.

Table 3 gives the designation of typical examples of Compound I, and Tables 4-1 and 4-2 show the structural formulae thereof. Table 5 gives physicochemical properties of the examples of Compound I or acid addition salts thereof.

Table 3

| Compound | Name |
|---|---|
| 1 | 5-[2-(Diethylamino)ethyl]amino-7-methoxy-5,11-dihydro[1]benzoxepino[3,4-b]pyridine |
| 2 | 5-[2-(Diethylamino)ethyl]amino-8-methoxy-5,11-dihydro[1]benzoxepino[3,4-b]pyridine |
| 3 | 5-[2-(Diethylamino)ethyl]amino-9-methoxy-5,11-dihydro[1]benzoxepino[3,4-b]pyridine |
| 4 | 5-[2-(Diethylamino)ethyl]amino-7-isopropoxy-5,11-dihydro[1]benzoxepino[3,4-b]pyridine |
| 5 | 5-[2-(Diethylamino)ethyl]amino-7,8-dimethoxy-5,11-dihydro[1]benzoxepino[3,4-b]pyridine |
| 6 | 5-[2-(Dimethylamino)ethyl]amino-7-methoxy-5,11-dihydro[1]benzoxepino[3,4-b]pyridine |
| 7 | 5-[3-(Dimethylamino)propyl]amino-7-methoxy-5,11-dihydro[1]benzoxepino[3,4-b]pyridine |
| 8 | 5-[3-(Diethylamino)propyl]amino-7-methoxy-5,11-dihydro[1]benzoxepino[3,4-b]pyridine |
| 9 | 5-[2-(Diisopropylamino)ethyl]amino-7-methoxy-5,11-dihydro[1]benzoxepino[3,4-b]pyridine |

Table 3 (continued)

| Compound | Name |
|---|---|
| 10 | 7-Methoxy-5-[2-(1-pyrrolidinyl)ethyl]amino-5,11-dihydro[1]benzoxepino[3,4-b]pyridine |
| 11 | 7-Methoxy-5-[2-(morpholino)ethyl]amino-5,11-dihydro[1]benzoxepino[3,4-b]pyridine |
| 12 | 5-[3-(Cyclohexylamino)propyl]amino-7-methoxy-5,11-dihydro[1]benzoxepino[3,4-b]pyridine |
| 13 | 5-[(1-Ethyl-2-pyrrolidinyl)methyl]amino-7-methoxy-5,11-dihydro[1]benzoxepino[3,4-b]pyridine |
| 14 | 5-[(1-Ethyl-2-pyrrolidinyl)methyl]amino-7-methoxy-5,11-dihydro[1]benzoxepino[3,4-b]pyridine |
| 15 | 5-(2-Aminoethyl)amino-7-methoxy-5,11-dihydro-[1]benzoxepino[3,4-b]pyridine |
| 16 | 5-(4-Methyl-1-piperadinyl)-7-methoxy-5,11-dihydro[1]benzoxepino[3,4-b]pyridine |
| 17 | 5-[2-(Diethylamino)ethyl]oxy-7-methoxy-5,11-dihydro[1]benzoxepino[3,4-b]pyridine |
| 18 | 5-[2-(Diethylamino)ethyl]amino-7-dimethylamino-5,11-dihydro[1]benzoxepino[3,4-b]pyridine |
| 19 | 5-[2-(Ethylamino)ethyl]amino-7-methoxy-5,11-dihydro[1]benzoxepino[3,4-b]pyridine |
| 20 | 5-{N-ethyl-N-[2-(diethylamino)]ethyl}amino-7-methoxy-5,11-dihydro[1]benzoxepino[3,4-b]pyridine |
| 21 | 5-[2-(2,6-Dimethylpiperidino)ethyl]amino-7-methoxy-5,11-dihydro[1]benzoxepino[3,4-b]pyridine |
| 22 | 5-[2-(Cis-2,5-dimethyl-1-pyrrolidino)ethyl]amino-7-methoxy-5,11-dihydro[1]benzoxepino[3,4-b]pyridine |

Table 3 (continued)

| Compound | Name |
|---|---|
| 23 | 5-[2-(Trans-2,5-dimethyl-1-pyrrolidino)ethyl]-amino-7-methoxy-5,11-dihydro[1]benzoxepino-[3,4-b]pyridine |
| 24 | 5-[2-(Diethylamino)ethyl]thio-7-methoxy-5,11-dihydro[1]benzoxepino[3,4-b]pyridine |
| 25 | 5-[(N,N-diethylamino)acetylamino-7-methoxy-5,11-dihydro[1]benzoxepino[3,4-b]pyridine |
| 26 | 5-Cyano-5-(3-diisopropylamino)propyl-5,11-dihydro[1]benzoxepino[3,4-b]pyridine |
| 27 | 5-Carbamoyl-5-(3-diisopropylamino)propyl-5,11-dihydro[1]benzoxepino[3,4-b]pyridine |
| 28 | 5-[2-(Diethylamino)ethyl]amino-7-hydroxy-5,11-dihydro[1]benzoxepino[3,4-b]pyridine |
| 29 | (E)-5-(3-diethylaminopropylidene)-7-methoxy-5,11-dihydro[1]benzoxepino[3,4-b]pyridine |
| 30 | (Z)-5-(3-diethylaminopropylidene)-7-methoxy-5,11-dihydro[1]benzoxepino[3,4-b]pyridine |
| 31 | (E)-5-(3-diethylaminopropylidene)-5,11-dihydro-[1]benzoxepino[3,4-b]pyridine |
| 32 | (Z)-5-(3-diethylaminopropylidene)-5,11-dihydro-[1]benzoxepino[3,4-b]pyridine |
| 33 | 5-[2-(diethylamino)ethyl]thio-5,11-dihydro[1]-benzoxepino[3,4-b]pyridine |
| 34 | 5-[2-diethylamino)ethyl]thio-7-methyl-5,11-dihydro[1]benzoxepinio[3,4-b]pyridine |

Table 4 - 1

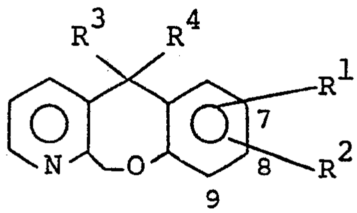

| Compound | $R^1$ | $R^2$ | $R^3$ | $R^4$ |
|---|---|---|---|---|
| 1 | 7 - OMe | H | 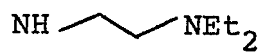 | H |
| 2 | 8 - OMe | " | " | " |
| 3 | 9 - OMe | " | " | " |
| 4 | 7 - OiPr | " | " | " |
| 5 | 7 - OMe | 8-OMe | " | " |
| 6 | " | H | 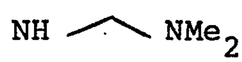 | " |
| 7 | " | " | 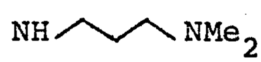 | " |
| 8 | " | " | 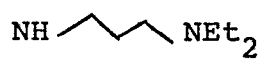 | " |
| 9 | " | " | 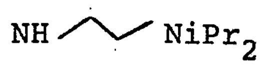 | " |
| 10 | " | " | 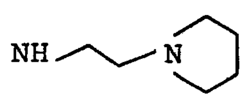 | " |
| 11 | " | " | 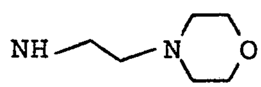 | " |
| 12 | " | " | 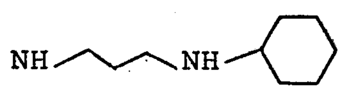 | " |
| 13 * | " | " | 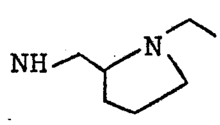 | " |
| 14 * | " | " | 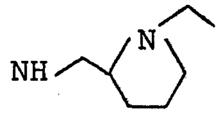 | " |

0027692

| Compound | $R^1$ | $R^2$ | $R^3$ | $R^4$ |
|---|---|---|---|---|
| 15 | 7 - OMe | H | 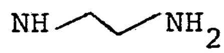 | H |
| 16 | " | " | 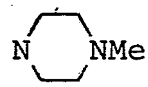 | " |
| 17 | " | " | 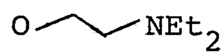 | " |
| 18 | 7 - $NMe_2$ | " | 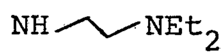 | " |
| 19 | 7 - OMe | " | 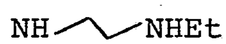 | " |
| 20 | " | " | 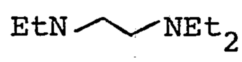 | " |
| 21 | " | " | 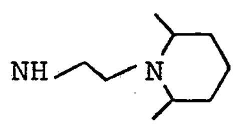 | " |
| 22 | " | " | 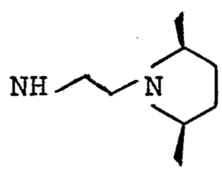 | " |
| 23 | " | " | 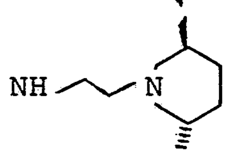 | " |
| 24 | " | " | 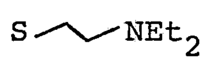 | " |
| 25 | " | " | 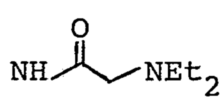 | " |
| 26 | H | " | $CH_2CH_2CH_2NiPr_2$ | CN |
| 27 | " | " | " | $CONH_2$ |
| 28 | 7 - OH | " | 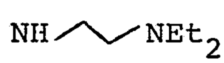 | H |
| 33 | H | " | 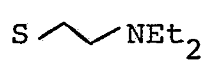 | " |
| 34 | Me | " | " | " |

* diastereomer

00270692

Table 4 - 2

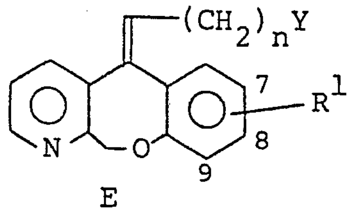

E

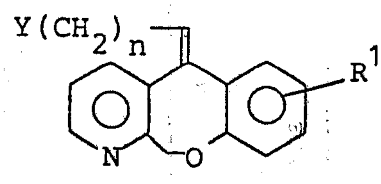

Z

| Compound | E or Z | $R^1$ | n | Y |
|---|---|---|---|---|
| 29 | E | 7 - OMe | 2 | $NEt_2$ |
| 30 | Z | " | " | " |
| 31 | E | H | " | " |
| 32 | Z | " | " | " |

Table 5

| Compound | IR Spectrum (Free base) ($cm^{-1}$) | H・NMR Spectrum (Free base) ($CDCl_3$, ppm) | M. P. (°C) | Elementary Analysis (%) Upper Calculated Lower Found C H N |
|---|---|---|---|---|
| 1 | (KBr) 3300, 2968, 1575, 1495, 1450, 1208, 1043 | 0.93(t,6H), 2.13-2.93(m,9H), 3.70(s,3H), 4.48(s,1H), 5.01 and 5.48(q,2H,AB type), 6.52-7.22(m,4H), 7.59(dd, 1H), 8.33(dd,1H) | Free base 85 - 86.5<br>1.5 Fumarate 128 - 129 | 1.5 fumarate<br>$C_{26}H_{33}O_8N_3$<br>60.57 6.45 8.15<br>60.59 6.51 8.23 |
| 2 | (Neat) 3300, 2950, 1615, 1450, 1160, 1040 | 0.93(t,6H), 2.07-2.74(m,9H), 3.71(s,3H), 4.45(s,1H), 5.00 and 5.88(q,2H,AB type), 6.38-7.27(m,4H), 7.55(dd, 1H), 8.36(dd,1H) | Monofumarate 132.5 - 134 | Monofumarate<br>$C_{24}H_{31}O_6N_3$<br>63.00 6.83 9.18<br>62.88 6.99 9.14 |
| 3 | (Neat) 3300, 2960, 1580, 1460, 1270, 1080 | 0.93(t,6H), 1.95-2.89(m,9H), 3.85(s,3H), 4.56(s,1H), 5.10 and 5.59(q,2H,AB type), 6.59-7.28(m,4H), 7.64(dd, 1H), 8.39(dd,1H) | Trihydrochloride dihydrate hemi-isopropanol 161 - 165 (Dec.) | Trihydrochloride dihydrate hemiisopropanol<br>$C_{20}H_{30}O_2N_3Cl_3\cdot 2H_2O\cdot 1/2C_3H_8O$<br>50.84 7.34 8.27<br>50.80 7.59 8.13 |

| | | | | |
|---|---|---|---|---|
| 4 | (Neat) 3300, 2975, 1580, 1500, 1210, 1040 | 0.93(t,6H), 1.30(d,6H), 2.14-2.84(m,9H), 4.18-4.65 (m,2H), 5.01 and 5.49(q,2H, AB type), 6.56-7.30(m,4H), 7.60(dd,1H), 8.35(dd,1H) | Trihydrochloride monohydrate<br>183 - 184 (Dec.) | Trihydrochloride monohydrate<br>$C_{22}H_{34}O_2N_3Cl_3 \cdot H_2O$<br>53.18 7.30 8.46<br>53.08 7.34 8.52 |
| 5 | (Neat) 3300, 2970, 1740, 1615, 1585, 1515, 1455 | 0.93(t,6H), 2.22-2.71(m,8H), 3.81(s,6H), 4.41(s,1H), 5.00 and 5.66(q,2H,AB type), 6.65(s,1H), 6.70(s,1H), 6.95-7.26(m,1H), 7.58(dd, 1H), 8.39(dd,1H) | Monofumarate<br>166 - 169 | Monofumarate<br>$C_{25}H_{33}O_7N_3$<br>61.59 6.82 8.62<br>61.70 6.98 8.58 |
| 6 | (Neat) 3300, 2950, 1570, 1495, 1200, 1040 | 2.08(s,6H), 2.12-2.76(m,4H), 2.85(s,1H), 3.70(s,3H), 4.44 (s,1H), 4.97 and 5.46(q,2H, AB type), 6.52-7.20(m,4H), 7.57(dd,1H), 8.32(dd,1H) | Trihydrochloride<br>190 - 191.5 | Trihydrochloride<br>$C_{18}H_{26}O_2N_3Cl_3$<br>51.14 6.20 9.94<br>50.94 6.38 9.85 |
| 7 | (Neat) 3300, 2930, 1500, 1265, 1208 | 1.46-2.79(m,6H), 2.15(s,6H), 3.72(s,3H), 4.46(s,1H), 5.01 and 5.50(q,2H,AB type), 6.59-7.76(m,5H), 8.37(dd,1H) | Trihydrochloride dihydrate<br>193 - 195 | Trihydrochloride dihydrate<br>$C_{19}H_{28}O_2N_3Cl_3 \cdot 2H_2O$<br>48.26 6.82 8.89<br>48.43 6.92 8.85 |

| | | | | |
|---|---|---|---|---|
| 8 | (Neat) 3250, 2950, 1575, 1495, 1200, 1040 | 0.94(t,6H), 1.37-1.97(m,2H), 2.16-2.77(m,9H), 3.70(s,3H), 4.44(s,1H), 5.00 and 5.50 (q,2H,AB type), 6.57-7.25(m,4H), 7.60(dd,1H), 8.37(dd,1H) | Trihydrochloride monohydrate 115 - 120 (Dec.) | Trihydrochloride monohydrate $C_{21}H_{32}O_2N_3Cl_3 \cdot H_2O$<br>52.23 7.10 8.70<br>52.23 7.40 8.51 |
| 9 | (KBr) 3300, 2950, 1575, 1440, 1260, 1040 | 0.90(d,12H), 2.34-3.09(m,7H), 4.46(s,1H), 5.01 and 5.45(q,2H,AB type), 6.53-7.22(m,4H), 7.58(dd,1H), 8.33(dd,1H) | Trihydrochloride hemihydrate monoisopropanol 166 - 169 (Dec.) | Trihydrochloride hemihydrate monoisopropanol $C_{22}H_{34}O_2N_3Cl_3 \cdot 1/2H_2O \cdot C_3H_8O$<br>54.79 7.91 7.67<br>54.43 7.83 7.60 |
| 10 | (Neat) 3300, 2945, 1570, 1495, 1200, 1040 | 1.46-2.75(m,12H), 2.83(s,1H), 3.69(s,3H), 4.43(s,1H), 4.97 and 5.47(q,2H,AB type), 6.53-7.20(m,4H), 7.57(dd,1H), 8.32(dd,1H) | Trihydrochloride monohydrate hemiisopropanol 172 - 175 (Dec.) | Trihydrochloride monohydrate hemiisopropanol $C_{20}H_{28}O_2N_3Cl_3 \cdot H_2O \cdot 1/2C_3H_8O$<br>51.97 6.90 8.47<br>52.00 6.83 8.37 |
| 11 | (KBr) 3420, 2940, 1580, 1500, 1270, 1040 | 2.05-2.81(m,8H), 2.93(s,1H), 3.43-3.79(m,4H), 3.73(s,1H), 4.40(s,1H), 4.98-5.46(q,2H,AB type), 6.53-7.22(m,4H), 7.59(dd,1H), 8.34(dd,1H) | Dihydrochloride monohydrate 159 - 162 (Dec.) | Dihydrochloride monohydrate $C_{20}H_{27}O_3N_3Cl_3 \cdot H_2O$<br>53.82 6.55 9.41<br>53.91 6.66 9.35 |

| | | | | |
|---|---|---|---|---|
| 12 | (Neat)<br>3300, 2925,<br>1580, 1500,<br>1210, 1050 | 0.69-2.92(m,17H), 2.26(s, 2H), 3.71(s,3H), 4.43(s,1H), 4.96 and 5.45(q,2H, AB type), 6.53-7.20(m,4H), 7.58(dd,1H), 8.32(dd,1H) | Trihydrochloride<br>236 - 237<br>(Dec.) | Trihydrochloride<br>$C_{23}H_{34}O_2N_3Cl_3$<br>56.27 6.98 8.56<br>56.14 7.18 8.27 |
| 13 | (Neat)<br>3300, 2950,<br>1570, 1495,<br>1200, 1040 | 0.85-3.36(m,15H), 3.69(s, 3H), 4.43(s,1H), 4.96-5.48 (q,2H,AB type), 6.51-7.19 (m,4H), 7.59(dd,1H), 8.31 (dd,1H) | Trihydrochloride monohydrate<br>185 - 187<br>(Dec.) | Trihydrochloride monohydrate<br>$C_{21}H_{30}O_2N_3Cl_3 \cdot H_2O$<br>52.45 6.71 8.74<br>52.62 6.72 8.74 |
| 14 | (Neat)<br>3300, 2950,<br>1570, 1495,<br>1200, 1040 | 0.80-3.29(m,15H), 3.68(s, 1H), 4.47(s,1H), 4.99-5.50 (q,2H,AB type), 6.53-7.25 (m,4H), 7.55(dd,1H), 8.33 (dd,1H) | Trihydrochloride monohydrate<br>160 - 162<br>(Dec.) | Trihydrochloride monohydrate<br>$C_{21}H_{30}O_2N_3Cl_3 \cdot H_2O$<br>52.45 6.71 8.74<br>52.42 6.92 8.73 |
| 15 | (Neat)<br>3300, 2925,<br>1575, 1490,<br>1200, 1040 | 2.31-2.95(m,4H), 3.71(s,3H), 4.41(s,1H), 4.97-5.46(q,2H, AB type), 6.56-7.22(m,4H), 7.57(dd,1H), 8.34(dd,1H) | Trihydrochloride monohydrate<br>170 - 173<br>(Dec.) | Trihydrochloride monohydrate<br>$C_{16}H_{22}O_2N_3Cl_3 \cdot H_2O$<br>46.56 5.86 10.18<br>46.71 6.00 9.99 |

| | | | | |
|---|---|---|---|---|
| 16 | ($CHCl_3$)<br>2930, 2800,<br>1495, 1265 | 2.20(s,3H), 2.35(s,8H),<br>3.70(s,3H), 3.90(s,1H),<br>4.91 and 6.33(q,2H,AB type),<br>6.61-7.63(m,5H), 8.43(dd,1H) | Free base<br>101 - 102 | Free base<br>$C_{19}H_{23}O_2N_3$<br>70.13 7.12 12.91<br>70.20 7.33 12.93 |
| 17 | (Neat)<br>2966, 1576,<br>1494, 1200,<br>1046 | 1.01(t,6H), 2.32-2.97(m,6H),<br>3.71(s,3H), 3.51-3.92(m,2H),<br>5.27(s,2H), 5.77(s,1H),<br>6.56-7.29(m,4H), 7.87(dd,<br>1H), 8.35(dd,1H) | Free base<br>Oily | —— |
| 18 | (Neat)<br>3300, 2970,<br>1615, 1510,<br>1210, 1040 | 0.94(t,6H), 2.10-2.85(m,9H),<br>2.88(s,3H), 4.45(s,1H), 5.01<br>and 5.49(q,2H,AB type),<br>6.47-6.75(m,2H), 6.93-7.28<br>(m,2H), 7.64(dd,1H), 8.35<br>(dd,1H) | Trihydrochloride<br>trihydrate<br>179 - 181<br>(Dec.) | Trihydrochloride trihydrate<br>$C_{21}H_{33}ON_4Cl_3 \cdot 3H_2O$<br>48.70 7.59 10.82<br>48.84 7.71 10.78 |
| 19 | (Neat)<br>3300, 2930,<br>1580, 1490,<br>1440, 1205 | 1.06(t,3H), 2.58(q,2H), 2.66<br>(s,4H), 3.73(s,3H), 4.46(s,<br>1H), 5.02 and 5.51(q,2H,AB<br>type), 6.61-7.30(m,4H), 7.63<br>(dd,1H), 8.39(dd,1H) | Trihydrochloride<br>hemihydrate<br>191 - 193<br>(Dec.) | Trihydrochloride<br>hemihydrate<br>$C_{18}H_{26}O_2N_3Cl_3 \cdot 1/2H_2O$<br>50.07 6.30 9.73<br>50.00 6.15 9.92 |

| | | | | |
|---|---|---|---|---|
| 20 | (Neat) 2955, 1580, 1495, 1210, 1050 | 0.64-1.24(m,9H), 2.02-2.95 (m,10H), 3.71(s,3H), 4.33 (s,1H), 4.91 and 6.27(q,2H, AB type), 6.59-7.29(m,4H), 7.55(dd,1H), 8.39(dd,1H) | Free base<br>Oily | —— |
| 21 | (Neat) 3350, 2930, 1580, 1500, 1210, 1050 | 0.81-2.81(m,19H), 3.73(s, 3H), 4.42(s,1H), 4.99 and 5.48(q,2H,AB type), 6.60-7.23(m,4H), 7.61(dd,1H), 8.36(dd,1H) | Trihydrochloride hemihydrate<br>204 - 205 | Trihydrochloride hemihydrate<br>$C_{23}H_{34}O_2N_3Cl_3 \cdot 1/2H_2O$<br>55.26 7.06 8.41<br>55.39 7.10 8.42 |
| 22 | (Neat) 3300, 2950, 1580, 1500, 1210, 1050 | 0.96(d,3H), 1.00(d,3H), 1.26-1.36(m,2H), 1.72-1.79 (m,2H), 2.50-2.67(m,6H), 3.77(s,3H), 4.48(s,1H), 5.05 and 5.53(q,2H,AB type), 6.75-6.80(m,2H), 7.06-7.16 (m,2H), 7.65(dd,1H), 8.43 (dd,1H) | Trihydrochloride<br>205 - 207<br>(Dec.) | Trihydrochloride<br>$C_{22}H_3O_2N_3Cl_3$<br>55.41 6.76 8.81<br>55.14 6.95 8.64 |

| | | | | |
|---|---|---|---|---|
| 23 | (Neat) 3300, 2950, 1580, 1500, 1210, 1050 | 0.88(d,3H), 0.89(d,3H), 1.23-1.37(m,2H), 1.89-1.99 (m,2H), 2.54-2.75(m,4H), 2.86-2.92(m,2H), 3.768 and 3.774(each s, total 3H), 4.47 and 4.49(each s, total 1H), 5.04 and 5.52(q,2H,AB type), 6.75-6.81(m,2H), 7.07-7.17(m,2H), 7.63 and 7.70(each dd, total 1H), 8.431 and 8.436(each dd, total 1H) | Trihydrochloride monohydrate 173 - 175 (Dec.) | Trihydrochloride monohydrate $C_{22}H_{32}O_2N_3Cl_3 \cdot H_2O$ 53.39 6.93 8.49 53.40 6.86 8.42 |
| 24 | (Neat) 2950, 1575, 1495, 1210, 1045 | 0.97(t,6H), 2.18-2.81(m,8H), 3.72(s,3H), 4.79(s,1H), 4.98 and 5.69(q,2H,AB type), 6.59 -7.27(m,4H), 7.54(dd,1H), 8.37(dd,1H) | Dihydrochloride 193 - 196 (Dec.) | Dihydrochloride $C_{20}H_{28}O_2N_2SCl_2$ 55.68 6.54 6.49 55.63 6.84 6.35 |
| 25 | (Neat) 3350, 2970, 1670, 1500, 1210, 1040 | 0.88(t,6H), 2.42(q,4H), 2.96 (s,2H), 3.73(s,3H), 5.00 and 5.44(q,2H,AB type), 5.94(d, 1H), 6.61-7.34(m,4H), 7.81 (dd,1H), 8.39(dd,1H), 8.63 (d,1H) | Dihydrochloride 205 - 207 | Dihydrochloride $C_{20}H_{27}O_3N_3Cl_2$ 56.08 6.35 9.81 56.26 6.53 9.82 |

| | | | | |
|---|---|---|---|---|
| 26 | (Neat)<br>2960, 2240, 1490, 1455, 1215 | 0.88(d,12H), 2.24-3.21(m, 6H), 5.00 and 5.46(q,2H,AB type), 7.05-7.44(m,4H), 7.67 -7.94(m,1H), 8.21(dd,1H), 8.43(dd,1H) | Free base<br>Oily | —— |
| 27 | Hemihydrate<br>(KBr)<br>3400, 2970, 1675, 1365, 1210 | Hemihydrate<br>0.96(d,6H), 1.01(d,6H), 2.50 (bs,4H), 2.80-3.44(m,2H), 5.04 and 5.37(q,2H,AB type), 6.01(bs,2H), 6.88-7.53(m, 5H), 7.72(dd,1H), 8.28(dd, 1H) | Hemihydrate<br>138 - 139 | Hemihydrate<br>$C_{22}H_{29}O_2N_3 \cdot 1/2H_2O$<br>70.18 8.03 11.16<br>70.02 8.05 11.09 |
| 28 | (KBr)<br>3260, 2800, 1580, 1480, 1295, 1050 | 0.86(t,6H), 2.33(q,4H), 2.43 (br,s,4H), 4.59(s,1H), 4.92 and 5.46(q,2H,AB type), 6.60-6.69(m,2H), 7.26(dd, 1H), 7.78(d,1H), 8.40(dd, 1H), 9.22(br,s,1H) | Free base<br>184 - 185.5 (Dec.)<br>Trihydrochloride<br>191 - 193 (Dec.) | Free base<br>$C_{19}H_{25}O_2N_3$<br>69.70 7.70 12.83<br>69.58 7.99 12.54<br>Trihydrochloride<br>$C_{19}H_{28}O_2N_3Cl_3$<br>52.24 6.46 9.62<br>52.24 6.63 9.48 |

| | | | | |
|---|---|---|---|---|
| 29 | (Neat) 2960, 1570, 1490, 1200, 1050 | 0.97(t,6H), 2.06-2.86(m,8H), 3.71(s,3H), 5.20(s,2H), 5.92 (t,1H), 6.60-7.27(m,4H), 7.61(dd,1H), 8.32(dd,1H) | Dihydrochloride 1/3 hydrate 180 - 181 (Dec.) | Dihydrochloride 1/3 hydrate $C_{21}H_{28}O_2N_2Cl_2 \cdot 1/3H_2O$ 60.43 6.92 6.71 60.78 7.11 6.57 |
| 30 | (Neat) 2960, 1570, 1490, 1200, 1050 | 0.95(t,6H), 2.05-2.73(m,8H), 3.70(s,3H), 5.21(s,2H), 6.02 (t,1H), 6.56-7.80(m,5H), 8.39(dd,1H) | Dihydrochloride 1/3 hydrate 203 - 204.5 (Dec.) | Dihydrochloride 1/3 hydrate $C_{21}H_{28}O_2N_2Cl_2 \cdot 1/3H_2O$ 60.43 6.92 6.71 60.31 6.92 6.52 |
| 31 | (Neat) 2955, 1600, 1480, 1210, 1040 | 0.98(t,6H), 2.20-2.77(m,8H), 5.25(s,2H), 5.86(t,1H), 6.89 -7.78(m,6H), 8.34(dd,1H) | Dihydrochloride 198 - 202 (Dec.) | Dihydrochloride $C_{20}H_{26}ON_2Cl_2$ 62.99 6.87 7.35 63.15 7.07 7.38 |
| 32 | (Neat) 2955, 1600, 1480, 1210, 1040 | 0.94(t,6H), 2.08-2.81(m,8H), 5.27(s,2H), 6.04(t,1H), 6.58-7.79(m,6H), 8.41(dd, 1H) | Dihydrochloride 2/3 hydrate 186 - 193 (Dec.) (containing 11.6% of Compound 31) | Dihydrochloride 2/3 hydrate $C_{20}H_{26}ON_2Cl_2 \cdot 2/3H_2O$ 61.06 7.00 7.12 61.00 7.00 7.03 |

| | | | | |
|---|---|---|---|---|
| 33 | (Neat) 2960, 1490, 1450, 1220, 1050 | ($CCl_4$) 0.97(t,6H), 2.23-2.73(m,8H), 4.83(s,1H), 5.00 and 5.94(q,2H,AB type), 6.50-7.37(m,5H), 7.45(dd,1H), 8.38(dd,1H) | — | — |
| 34 | — | 0.96(t,6H), 2.24(s,3H), 2.21-2.63(m,8H), 4.81(s,1H), 5.00 and 5.85(q,2H,AB type), 6.93-7.28(m,4H), 7.51(dd, 1H), 8.37(dd,1H) | — | — |

Example

Examples and Compound Production Examples of the present invention are shown below.

Example 1 Tablet

A tablet comprising the following components was prepared in a conventional manner.

| | |
|---|---|
| 1.5 fumarate of Compound 1 | 20 mg |
| Lactose | 60 mg |
| Potato starch | 30 mg |
| Polyvinyl alcohol | 2 mg |
| Magnesium stearate | 1 mg |
| Tar pigment | q.s. |

Example 2 Powder

A powder comprising the following components was prepared in a conventional manner.

| | |
|---|---|
| Trihydrochloride monohydrate of Compound 4 | 20 mg |
| Lactose | 280 mg |

Example 3 Injection

A distilled water for injection was added to 100 mg of trihydrochloride monohydrate of Compound 8, until the total volume was 20 ml. Then, an injection preparation was prepared in a conventional manner.

Compound production example 1

In this example, 6.00 g of 5-hydroxy-7-methoxy-5,11-dihydro[1]benzoxepino[3,4-b]pyridine obtained in Compound production example 36 was dissolved in 100 ml of dichloromethane and 2.7 ml of thionyl chloride was added thereto under ice cooling. The resultant solution was stirred under ice cooling for 30 minutes and at room temperature for one hour. The reaction mixture was concentrated under reduced pressure to dryness and the residue was dissolved in 30 ml of dichloromethane. To the solution

was added under ice cooling a solution of 14.50 g of N,N-diethylethylenediamine dissolved in 120 ml of dichloromethane. After stirring the reaction mixture under ice cooling for 30 minutes and at room temperature for one hour, 100 ml of dichloromethane was added thereto. The mixture was washed with water, dried over anhydrous sodium sulfate, filtered and concentrated under reduced pressure. The residue was subjected to column chromatography on silica gel using ethylacetate / triethylamine = 20 / 1 as an eluent to obtain 7.21 g of Compound 1 as crystals.

One gram of Compound 1 was reacted with 0.51 g of fumaric acid in isopropanol to obtain crystals which was recrystallized from isopropanol to obtain 1.07 g of 1.5 fumarate of Compound 1.

<u>Compound production examples 2 - 20</u>

The similar procedures as in Compound production example 1 were repeated except that the raw materials shown in Table 6 were used instead of 6.00 g of 5-hydroxy-7-methoxy-5,11-dihydro[1]benzoxepino[3,4-b]pyridine and 14.50 g of N,N-diethylethylenediamine in Compound production example 1 to obtain Compounds 2 - 20 in Table 6.

Table 6

| Compound Production Example | Raw Material | | Product | |
|---|---|---|---|---|
| | Compound | Used Amount (g) | Compound No. | Obtained Amount (g) |
| 2 | 5-Hydroxy-8-methoxy-5,11-dihydro[1]benzoxepino-[3,4-b]pyridine<br>N,N-diethylethylene-diamine | 2.00<br>4.80 | 2 | 2.35 |
| 3 | 5-Hydroxy-9-methoxy-5,11-dihydro[1]benzoxepino-[3,4-b]pyridine<br>N,N-diethylethylene-diamine | 0.97<br>2.30 | 3 | 0.58 |
| 4 | 5-Hydroxy-7-isopropoxy-5,11-dihydro[1]benzoxepino[3,4-b]pyridine<br>N,N-diethylethylene-diamine | 1.94<br>4.15 | 4 | 2.32 |
| 5 | 7,8-Dimethoxy-5-hydroxy-5,11-dihydro[1]benzoxepino[3,4-b]pyridine<br>N,N-diethylethylene-diamine | 1.35<br>2.90 | 5 | 1.43 |
| 6 | 5-Hydroxy-7-methoxy-5,11-dihydro[1]benzoxepino-[3,4-b]pyridine<br>N,N-dimethylethylene-diamine | 2.00<br>3.60 | 6 | 2.06 |
| 7 | 5-Hydroxy-7-methoxy-5,11-dihydro[1]benzoxepino-[3,4-b]pyridine<br>3-Diethylaminopropyl-amine | 2.00<br>4.19 | 7 | 1.40 |

| Compound Production Example | Raw Material | | Product | |
|---|---|---|---|---|
| | Compound | Used Amount (g) | Compound No. | Obtained Amount (g) |
| 8 | 5-Hydroxy-7-methoxy-5,11-dihydro[1]benzoxepino-[3,4-b]pyridine<br>3-Diethylaminopropylamine | 2.00<br>5.35 | 8 | 2.29 |
| 9 | 5-Hydroxy-7-methoxy-5,11-dihydro[1]benzoxepino-[3,4-b]pyridine<br>N,N-diisopropylethylenediamine | 2.00<br>5.90 | 9 | 2.44 |
| 10 | 5-Hydroxy-7-methoxy-5,11-dihydro[1]benzoxepino-[3,4-b]pyridine<br>1-(2-Aminoethyl)-pyrrolidine | 2.00<br>4.70 | 10 | 1.79 |
| 11 | 5-Hydroxy-7-methoxy-5,11-dihydro[1]benzoxepino-[3,4-b]pyridine<br>N-(2-aminoethyl)-morphorine | 2.00<br>5.35 | 11 | 2.18 |
| 12 | 5-Hydroxy-7-methoxy-5,11-dihydro[1]benzoxepino-[3,4-b]pyridine<br>N-(3-aminopropyl)cyclohexylamine | 2.00<br>6.42 | 12 | 2.83 |
| 13<br>14 | 5-Hydroxy-7-methoxy-5,11-dihydro[1]benzoxepino-[3,4-b]pyridine<br>2-(Aminoethyl)-1-ethylpyrrolidine | 4.00<br>10.60 | 13<br>14 | 2.14<br>1.90 |

| Compound Production Example | Raw Material | | Product | |
|---|---|---|---|---|
| | Compound | Used Amount (g) | Compound No. | Obtained Amount (g) |
| 15 | 5-Hydroxy-7-methoxy-5,11-dihydro[1]benzoxepino-[3,4-b]pyridine<br>Ethylenediamine | 2.00<br>4.90 | 15 | 1.58 |
| 16 | 5-Hydroxy-7-methoxy-5,11-dihydro[1]benzoxepino-[3,4-b]pyridine<br>N-methylpiperizine | 1.50<br>3.10 | 16 | 1.13 |
| 17 | 5-Hydroxy-7-methoxy-5,11-dihydro[1]benzoxepino-[3,4-b]pyridine<br>2-Diethylaminoethanol | 2.00<br>4.80 | 17 | 2.24 |
| 18 | 7-Dimethylamino-5-hydroxy-5,11-dihydro[1]-benzoxepino[3,4-b]-pyridine<br>N,N-Diethylethylene-diamine | 1.57<br>3.60 | 18 | 1.14 |
| 19 | 5-Hydroxy-7-methoxy-5,11-dihydro[1]benzoxepino-[3,4-b]pyridine<br>N-ethylethylenediamine | 5.00<br>9.08 | 19 | 2.05 |
| 20 | 5-Hydroxy-7-methoxy-5,11-dihydro[1]benzoxepino-[3,4-b]pyridine<br>N,N,N'-triethylethylene-diamine | 2.00<br>5.90 | 20 | 2.19 |

Compound production example 21

In this example, 2.00 g of 5-amino-7-methoxy-5,11-dihydro[1]benzoxepino[3,4-b]pyridine, 2.60 g of hydrochloride of 2-(2,6-dimethylpiperidino)ethylchloride, 3.5 ml of triethylamine and a catalytic amount of sodium iodide were refluxed in 60 ml of toluene for 12 hours. After cooling, the reaction mixture was washed with water and then saturated aqueous sodium chloride solution, dried over anhydrous sodium sulfate, filtered and concentrated under reduced pressure. The residue was subjected to column chromatography on silica gel using ethylacetate / triethylamine = 10 / 1 as eluent to obtain 2.11 g of Compound 21.

The product was treated with isopropanol hydrogen chloride solution in isopropanol to obtain trihydrochloride 1/2 hydrate of Compound 21.

Compound production examples 22 and 23

The similar procedures as in Compound production example 21 were repeated by using 4.80 g of 5-amino-7-methoxy-5,11-dihydro[1]benzoxepino[3,4-b]pyridine and 6.0 g of hydrochloride of 2-(2,5-dimethyl-1-piperidinyl)ethylchloride to obtain a mixture of Compounds 22 and 23. The mixture was subjected to column chromatography on silica gel (eluent: n-hexane : ethylacetate : triethylamine = 5 : 10 : 1) to obtain 2.79 g of Compound 22 and 1.51 g of Compound 23. Hydrochlorides of Compound 22 and Compound 23 were prepared according to the similar manner as in Compound production example 21.

Compound production example 24

A mixture of 2.00 g of 5-hydroxy-7-methoxy-5,11-dihydro[1]benzoxepino[3,4-b]pyridine and 2.6 ml of trifluoro acetic acid anhydride was stirred at room temperature under nitrogen gas atmosphere for 30 minutes. To the mixture was added 4.20 g of 2-diethylaminoethanethiol hydrochloride and the mixture was stirred at 100°C for 12 hours. After allowing the mixture to stand for cooling, the reaction

solution was poured into ice water.

After nutralizing the solution with aqueous sodium hydrooxide solution, organic solvent layer was washed with water, dried over anhydrous sodium sulfate, filtered and concentrated under reduced pressure. The residue was subjected to column chromatography on silica gel (eluent: n-hexane : ethylacetate : triethylamine = 10 : 5 : 1) to obtain 2.74 g of Compound 24 as oily matter.

Dihydrochloride of Compound 24 was prepared according to the similar manner as in Compound production example 21.

Compound production example 25

In this example, 2.00 g of 5-amino-7-methoxy-5,11-dihydro[1]benzoxepino[3,4-b]pyridine and 1.2 ml of triethylamine were dissolved in 50 ml of ethylether and 0.93 g of chloroacetyl chloride was dropwise added thereto under ice cooling. After stirring the solution under ice cooling for one hour, ethylacetate was added thereto. The mixture was washed with water and then saturated aqueous sodium chloride solution, dried over anhydrous sodium sulfate and filtered, and the solvent was evaporated under reduced pressure. To the residue were added 70 ml of toluene and 3.0 g diethylamine and the mixture was refluxed for 3 hours. Then, ethyl acetate was added thereto. The mixture was washed with water and then saturated aqueous sodium chloride solution, dried over anhydrous sodium sulfate and filtered, and the filtrate was concentrated under reduced pressure. The residue was subjected to column chromatography on silica gel (eluent: ethyl acetate : n-hexane : triethylamine = 10 : 5 : 1) to obtain 2.35 g of Compound 25 as oily matter.

Dihydrochloride of Compound 25 was prepared according to the similar manner as in Compound production example 21.

Compound production example 26

In this example, 3.00 g of 5-hydroxy-5,11-dihydro[1]benzoxepino[3,4-b]pyridine was suspended in 60 ml of dichloromethane and 2.0 ml of thionyl chloride was added thereto under ice cooling. The mixture was subjected to reaction under ice cooling for 30 minutes and at room temperature for 1 hour. The mixture was concentrated to dryness under reduced pressure, and 60 ml of acetonitrile and 4.69 g of silver cyanide were added thereto and the mixture was refluxed for one and a half hours. After completion of the reaction, the mixture was filtered and ethyl acetate and water were added to the filtrate. After shaking, an aqueous layer was discarded and the organic solvent layer was washed with saturated aqueous sodium chloride solution, dried over anhydrous sodium sulfate and filtered and concentrated under reduced pressure. The residue was subjected to column chromatography on silica gel using n-hexane / ethyl acetate = 4 / 1 as eluent to obtain 1.70 g of 5-cyano-5,11-dihydro[1]benzoxepino[3,4-b]-pyridine.

IR(KBr): 2900, 2250, 1490, 1450

$^1$H-NMR ($CDCl_3$): 5.12 and 5.80(q, 2H, AB type), 5.31 (s, 1H), 6.89-7.56(m, 5H), 7.85(dd, 1H), 8.39 (dd, H).

Then, 1.50 g of 5-cyano-5,11-dihydro[1]benzoxepino[3,4-b]pyridine obtained above and 0.31 g of sodium amide were heated in 25 ml of toluene at 100°C for one hour. 1.7 g of 3-(N,N-diisopropylamino)propyl chloride were added thereto and the mixture was refluxed for 7 hours. After allowing the mixture to stand for cooling, 20 ml of water was added thereto and the mixture was extracted with ethyl acetate. The extract was washed with saturated aqueous sodium chloride solution, dried over anhydrous sodium sulfate and filtered and the filtrate was concentrated under reduced pressure. The residue was subjected to column chromatography on silica gel (eluent: n-hexane : ethyl acetate : triethylamine = 10 : 2 : 1) to obtain 2.15 g of Compound 26.

Compound production example 27

In this example, 1.25 g of Compound 26 obtained in Compound production example 26 was heated at 90 - 95°C in 3.6 ml of concentrated sulfuric acid with stirring for 2 hours. After allowing the mixture to stand for cooling, the mixture was poured on ice and was alkalinized with aqueous sodium hydroxide solution. The mixture was extracted with ethyl acetate. The organic solvent layer was washed with water and then saturated aqueous sodium chloride solution, dried over anhydrous sodium sulfate and filtered and the filtrate was concentrated to dryness under reduced pressure to obtain 0.96 g of 1/2 hydrate of Compound 27 as crystals.

Compound production example 28

To a solution obtained by dissolving 2.18 g of Compound 1 in 60 ml of dichloromethane was dropwise added a solution obtained by dissolving 1.2 ml of boron tribromide in 12 ml of dichloromethane at -60°C.

After completion of the addition, the temperature of the mixture was gradually brought back to room temperature and the mixture was stirred at room temperature over night. After extracting the reaction solution with water, the pH of the mixture was adjusted to 9 with aqueous sodium hydroxide solution. After adding dichloromethane, the mixture was stirred to deposit crystals. The crystals were separated by filtration and dried to obtain 1.00 g of Compound 28. Trihydrochloride of Compound 28 was prepared according to the similar manner as in Compound production example 21.

Compound production examples 29 and 30

In 300 ml of tetrahydrofuran was suspended 45.0 g of hydrobromate of 3-diethylaminopropyl-triphenylphosphonium bromide and 116 ml of 1.5M n-butyllithium was dropwise added thereto under ice cooling. After the addition, the

mixture was stirred at room temperature for two and a half hours. To the mixture was dropwise added under ice cooling a solution obtained by dissolving 10.0 g of 5-oxo-7-methoxy-5,11-dihydro[1]benzoxepino[3,4-b]pyridine in 160 ml of tetrahydrofuran. After stirring the reaction solution at room temperature overnight, the solution was concentrated under reduced pressure and ethyl acetate was added to the residue. The mixture was washed with water and then with saturated aqueous sodium chloride solution, dried over anhydrous sodium sulfate, filtered and concentrated under reduced pressure. The residue was subjected to column chromatography on silica gel (eluent: n-hexane : ethylacetate : triethylamine = 10 : 5 : 1) to obtain 4.73 g of Compound 29 and 5.21 g of Compound 30.

According to the similar manner to in Compound production example 21, trihydrochloride 1/3 hydrate of Compound 29 and trihydrochloride 1/3 hydrate of Compound 30 were obtained.

Compound production examples 31 and 32

The similar procedures as in Compound production examples 29 and 30 were repeated using 6.33 g of 5-oxo-5,11-dihydro[1]benzoxepino[3,4-b]pyridine and 32.6 g of hydrobromate of 3-diethylaminopropyl-triphenylphosphonium bromide to obtain 3.17 g of Compound 31 and 2.80 g of Compound 32 (containing a small amount of Compound 31).

According to the similar manner to in Compound production example 21, dihydrochloride of Compound 31 and dihydrochloride 2/3 hydrate of Compound 32 (containing 12% Compound 31) were obtained.

Compound production example 33

In this example, 4.0 g of 5-oxo-5,11-dihydro[1]-benzoxepino[3,4-b]pyridine was dissolved in 30 ml of concentrated sulfuric acid. To the solution was dropwise added a mixture of 2 g of concentrated nitric acid and 1 ml of concentrated sulfuric acid at -30°C. After the addition,

the temperature of the mixture was gradually brought back to room temperature, and the mixture was poured into ice water and was made alkaline with aqueous sodium hydroxide solution. After separating crystals by filtration, the crystals were washed with ether and dried to obtain 4.2 g of 7-nitro-5-oxo-5,11-dihydro[1]benzoxepino[3,4-b]pyridine.

Melting point: 205 - 207°C (Decomposition)
IR (KBr disk): 1660, 1610, 1580, 1518, 1340, 1080 $cm^{-1}$
$^{1}H$-NMR (DMSO-$d_6$): 5.58(s,2H), 7.42(d,1H), 7.67(dd, 1H), 8.25(dd, 1H), 8.43(dd, 1H), 8.84(dd, 1H), 8.90(d, 1H)

Then, 4.0 g of 7-nitro-5-oxo-5,11-dihydro[1]-benzoxepino[3,4-b]pyridine was suspended in a mixed solution of 150 ml of ethanol and 40 ml of water. To the suspension were added 4.7 ml of concentrated hydrochloric acid solution and 0.4 g of 10% Pd-c and hydrogen gas was fed to the mixture at room temperature with stirring for one hour. After the reaction mixture was filtered, the filtrate was concentrated to dryness under reduced pressure and the residue was dissolved in water. The mixture was made alkaline with aqueous sodium hydroxide solution and the deposited crystals are separated by filtration, and dried to obtain 3.1 g of 7-amino-5-oxo-5,11-dihydro[1]benzoxepino-[3,4-b]pyridine.

Melting point: 149 - 151.5°C
IR (KBr disk): 3400, 3330, 1650, 1618, 1490, 1320 $cm^{-1}$
$^{1}H$-NMR ($CDCl_3$ + DMSO-$d_6$): 4.78(brs, 2H), 5.19(s, 2H), 6.84(d, 2H), 7.23-7.59(m, 2H), 8.22(dd, 1H), 8.62(dd, 1H)

Then, 2.6 g of 7-amino-5-oxo-5,11-dihydro[1]-benzoxepino[3,4-b]pyridine was dissolved in a mixed solvent of 80 ml of ethanol and 30 ml of tetrahydrofuran. To the solution was added 0.3 g of sodium boron hydride at room temperature and the mixture was stirred overnight. The reaction mixture was poured into water and crystals were separated by filtration, dried to obtain 2.0 g of 7-amino-5-hydroxy-5,11-dihydro[1]benzoxepino[3,4-b]pyridine.

Melting point: 176 - 178°C
IR (KBr): 3380, 1580, 1500, 1200, 1050 $cm^{-1}$
$^1H$-NMR ($DMSO-d_6$ + $CDCl_3$): 4.51(bs, 2H), 4.94 and 5.23 (q, 2H, AB type), 5.73-6.89(m, 5H), 7.10(dd, 1H), 7.93(dd, H), 8.23(dd, 1H)

Then, 2.0 g of 7-amino-5-hydroxy-5,11-dihydro[1]-benzoxepino[3,4-b]pyridine was dissolved in 70 ml of methanol and the solution was made weak acid with hydrochloric acid. To the solution were added 2.7 g of sodium cyanoborohydride and 35% aqueous formaldehyde solution and the mixture was stirred at room temperature for one hour. After the concentration of the mixture under reduced pressure, water was added thereto and the mixture was made alkaline with aqueous sodium hydroxide solution. The mixture was extracted with ethyl acetate, washed with water, dried over anhydrous sodium sulfate and filtered, and the filtrate was concentrated to dryness under reduced pressure to obtain crude crystals. The product is recrysrallized from isopropanol to obtain 1.7 g of 7-dimethylamino-5-hydroxy-5,11-dihydro[1]benzoxepino[3,4-b]pyridine.

Melting point: 154 - 157°C
IR (KBr): 3150, 1615, 1580, 1510, 1220, 1050 $cm^{-1}$
$^1H$-NMR ($DMSO-d_6$ + $CDCl_3$): 2.80(s, 6H), 5.03 and 5.26 (q, 2H, AB type), 5.89(bs, 1H), 6.14(s, 1H), 6.33-7.26(m, 4H), 7.94(dd, 1H), 8.22(dd, 1H)

Compound production example 34

In this example, 5.0 g of 5-hydroxy-7-methoxy-5,11-dihydro[1]benzoxepino[3,4-b]pyridine was dissolved in 120 ml of dichloromethane and 1.6 ml of thionyl chloride was added thereto under ice cooling. The mixture was stirred under ice cooling for 30 minutes and at room temperature for one hour. The reaction solution was concentrated to dryness under reduced pressure and the residue was dissolved in 100 ml of dichloromethane. The solution was added dropwise under ice cooling to 300 ml of dichloromethane saturated with ammonia gas and the mixture was stirred at

room temperature for one hour. The mixture was filtered and the filtrate was washed with water, dried over anhydrous sodium sulfate and filtered. The organic solvent was evaporated under reduced pressure to obtain 4.9 g of 5-amino-7-methoxy-5,11-dihydro[1]benzoxepino[3,4-b]pyridine as oily matter

IR (neat): 3370, 1580, 1500, 1210, 1040 $cm^{-1}$

$^1$H-NMR ($CDCl_3$): 2.33(s, 2H), 3.72(s, 3H), 4.86(s, 1H), 5.07 and 5.39(q, 2H, AB type), 6.577(m, 4H), 7.67(dd, 1H), 8.36(dd, 1H)

Compound production example 35

In this example, 100.0 g of 2-(4-methoxyphenoxy)-methylpyridine-3-carboxylic acid was suspended in 1.5 ℓ of 1,1,2,2-tetrachloroethane and 136 ml of trifluoroacetic acid anhydride was added thereto at room temperature. After stirring the mixture for 40 minutes, 38 ml of complex of boron trifluoride and ethylether was added thereto and the mixture was stirred at 100 - 110°C for 4 hours. After allowing the mixture to stand for cooling, the mixture was poured into ice water and aqueous sodium hydroxide solution was added thereto to make the aqueous layer alkaline. After shaking, the aqueous layer was discarded, and the organic solvent layer was dried over anhydrous sodium sulfate and filtered. The filtrate was concentrated under reduced pressure. The residue was subjected to column chromatography on silica gel using n-hexane / ethyl acetate = 2 / 1 as eluent to obtain 67.0 g of 7-methoxy-5-oxo-5,11-dihydro[1]benzoxepino[3,4-b]pyridine as crystals.

Melting point: 79 - 80.5°C (isopropylether)

IR (KBr disk): 1643, 1610, 1489, 1300 $cm^{-1}$

$^1$H-NMR ($CDCl_3$): 3.78(s, 3H), 5.24(s, 2H), 6.93-7.70 (m, 4H), 8.25(dd, 1H), 8.63(dd, 1H)

Compound production example 36

In a mixed solvent of 200 ml of ethanol and 100 ml of tetrahydrofuran was dissolved 10.0 g of 7-methoxy-5-oxo-5,11-dihydro[1]benzoxepino[3,4-b]pyridine obtained in Compound production example 35 and 1.0 g of sodium boron hydride was added thereto under ice cooling. The mixture was stirred at room temperature for three hours and concentrated under reduced pressure. Ethyl acetate and water were added to the residue and the mixture was shaken. The aqueous layer was discarded, and the organic solvent layer was washed with saturated aqueous sodium chloride solution, dried over anhydrous sodium sulfate and filtered. The filtrate was concentrated to dryness under reduced pressure and the resultant crude crystals were recrystallized from toluene to obtain 7.1 g of 5-hydroxy-7-methoxy-5,11-dihydro[1]benzoxepino[3,4-b]pyridine as white crystals.

Melting point: 128 - 130.5°C

IR (KBr disk): 3150, 1590, 1490, 1435, 1200 $cm^{-1}$

$^1H$-NMR ($CDCl_3$ + DMSO-$d_6$): 3.69(s, 3H), 5.07 and 5.30 (q, 2H, AB type), 5.92(d, 1H), 6.18(d, 1H), 6.48-7.28(m, 4H), 7.94(dd, 1H), 8.22(dd, 1H)

Compound production example 37

In 15 ml of dichloromethane was suspended 1.00 g 5-hydroxy-5,11-dihydro[1]benzoxepino[3,4-b]pyridine and 0.45 ml of thionyl chloride was added thereto under ice cooling. The mixture was stirred under ice cooling for 30 minutes and then at room temperature for one hour.

The reaction solution was concentrated to dryness under reduced pressure and the residue was dissolved in 15 ml of N,N-dimethylformamide. Then, 1.60 g 2-diethylaminoethanethiol hydrochloride and 3.20 g of potassium carbonate were added thereto and the mixture was stirred at room temperature for 6 hours. The resultant mixture was added to water and the mixture was extracted with ethyl acetate. The organic solvent layer was washed with water, and then saturated aqueous sodium chloride solution, dried over

anhydrous sodium sulfate and filtered and the filtrate was concentrated under reduced pressure. The residue was subjected to column chromatography on silica gel (eluent: n-hexane : ethyl acetate : triethylamine = 15 : 5 : 1) to obtain 0.74 g of Compound 33 as oily matter.

Compound production example 38

The similar procedures as in Compound production example 37 were repeated using 2.00 g of 5-hydroxy-7-methyl-5,11-dihydro[1]benzoxepino[3,4-b]pyridine and 4.50 g of 2-diethylaminoethane thiol hydrochloride to obtain 2.11 g of Compound 34 as oily matter.

0270692

## Scope of Claims

1. An antiarrhythmic agent containing [1]benzoxepino [3,4-b]pyridine derivatives represented by the formula (I) and a pharmacologically acceptable acid addition salt thereof;

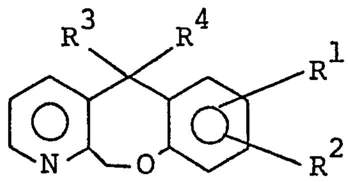

( I )

In the formula,

a) $R^1$ represents lower alkoxy, halogenated lower alkyl, halogenated lower alkoxy, hydroxy, lower alkylamino, di-lower alkylamino, cycloalkylamino, lower alkanoylamino, carboxyamino or lower alkoxycarbonylamino,
$R^2$ represents hydrogen,
$R^3$ represents $Z\text{-}N\text{-}(CH_2)_n\text{-}Y$ or 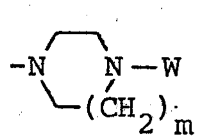

wherein Y represents amino, lower alkylamino, di-lower alkylamino, cycloalkylamino, heterocyclic group containing nitrogen atom or lower alkyl-substituted heterocyclic group containing nitrogen,
Z represents hydrogen, lower alkyl or lower alkanoyl
n represents 1, 2 or 3,
W represents hydrogen or lower alkyl, and
m represents 0, 1 or 2, and
$R^4$ represents hydrogen or

b) $R^1$ represents lower alkoxy or halogenated lower alkyl,
$R^2$ represents hydrogen,
$R^3$ represents $-NHCOCH_2Y$ wherein Y has the same meaning as previously defined, and
$R^4$ represents hydrogen, or

c) $R^1$ and $R^2$ simultaneously represent lower alkoxy,

$R^3$ represents $Z-N-(CH_2)_n-Y$, 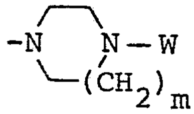 or $-X-(CH_2)_n-Y$

wherein n, Y, Z, m and W have the same meanings as previously defined, and X represents oxygen or sulfur, and

$R^4$ represents hydrogen or

d) $R^1$ represents hydrogen, lower alkyl, halogen, halogenated lower alkyl, lower alkoxy, halogenated lower alkoxy, hydroxy, lower alkylamino, di-lower alkylamino, cycloalkylamino, lower alkanoylamino, carboxyamino or lower alkoxycarbonylamino,

$R^2$ represents hydrogen,

With respect of $R^3$ and $R^4$

(1) $R^3$ represents $-X-(CH_2)_n-Y$ wherein n, X and Y have the same meanings as previously defined, and $R^4$ represents hydrogen;

(2) $R^3$ and $R^4$, taken together, are $=CH(CH_2)_nY$ wherein n and Y have the same meanings as previously defined, or

(3) $R^3$ represents $-(CH_2)_{n+1}-Y$ wherein n and Y have the same meanings as previously defined and $R^4$ represents cyano or carbamoyl.

2. Antiarrhythmic agent according to claim 1, wherein said [1]benzoxepino[3,4-b]pyridine derivative is a compound represented by formula (I-1):

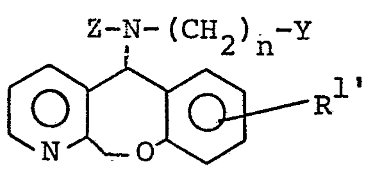

( I-1)

wherein Z, Y and n have the same meanings as previously defined in formula (I) and $R^{1'}$ is lower alkoxy.

3. Antiarrhythmic agent according to claim 2, wherein $R^{1'}$ is bound to 7-position.

4. A compound represented by formula (I-11) and a pharmacologically acceptable acid addition salt thereof.

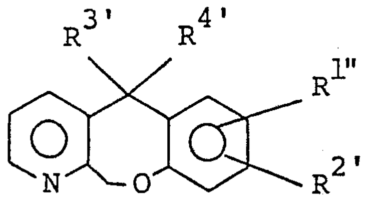

(I-11)

In the formula:

a) $R^{1''}$ represents halogenated lower alkoxy, hydroxy, lower alkylamino, di-lower alkylamino, cycloalkylamino, lower alkanoylamino, carboxyamino or lower alkoxycarbonylamino;

$R^{2'}$ represents hydrogen

$R^{3'}$ represents $Z-N-(CH_2)_n-Y$ or 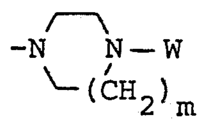

wherein Y represents amino, lower alkylamino, di-lower alkylamino, cycloalkylamino, heterocyclic group containing nitrogen or lower alkyl-substituted heterocyclic group containing nitrogen,

Z represents hydrogen, lower alkyl or lower alkanoyl,

n represents 1, 2 or 3,

W represents hydrogen or lower alkyl,

m represents 0, 1 or 2, and

$R^{4'}$ represents hydrogen,

b) $R^{1''}$ and $R^{2'}$, taken together, are lower alkoxy,

$R^{3'}$ represents $Z-N-(CH_2)_n-Y$, 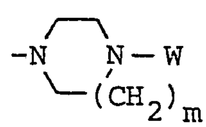 or

$-X-(CH_2)_n-Y$ wherein n, Y, Z, m and W have the same meanings as previously defined and X is oxygen atom or sulfur and

$R^{4'}$ represents hydrogen or

c) $R^{1''}$ represents hydrogen, lower alkyl, halogen, halogenated lower alkyl, lower alkoxy, halogenated lower alkoxy, hydroxy, lower alkylamino, di-lower alkylamino, cycloalkylamino, lower alkanoylamino, carboxyamino or lower alkoxycarbonylamino,
$R^{2'}$ represents hydrogen,
With respect to $R^{3'}$ and $R^{4'}$,

(1) $R^{3'}$ represents $-X-(CH_2)_n-Y$ wherein n, X and Y have the same meanings as previously defined, and $R^{4'}$ represents hydrogen

(2) $R^{3'}$ and $R^{4'}$, taken together, are $=CH_2(CH_2)_nY$ wherein n and Y have the same meanings as previously defined or

(3) $R^{3'}$ represents $-(CH_2)_{n+1}-Y$ wherein n and Y have the same meanings as previously defined and $R^{4'}$ represents cyano or carbamoyl.

5. The compound or pharmacologically acceptable acid addition salt according to claim 4, wherein $R^{1''}$ is lower alkoxy, $R^{2''}$ is hydrogen, $R^{3''}$ is $NHCH_2CH_2N(C_2H_5)_2$,

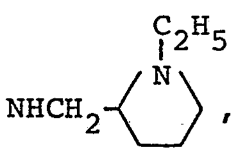 , 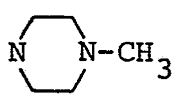 or 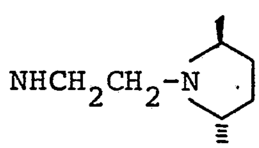 and $R^{4'}$ is hydrogen.

6. 5-[2-(diethylamino)ethyl]amino-7-methoxy-5,11-dihydro[1]benzoxepino[3,4-b]pyridine and a pharmacologically acceptable acid addition salt thereof.